# EUROPEAN PATENT APPLICATION

(11) **EP 4 368 984 A1**
(43) Date of publication of application: **15.05.2024**
(21) Application number: 22837478.1
(22) Date of filing: 22.06.2022
(51) Int. Cl.: G01N 27/62, H01J 49/16

(54) **MICROORGANISM MASS SPECTROMETRY METHOD**

(30) Priority: 08.07.2021 JP 2021113657
(71) Applicant: Shimadzu Corporation, Nakagyo-ku, Kyoto-shi, Kyoto 604-8511 (JP)
(72) Inventor: TERAMOTO, Kanae, Kyoto-shi, Kyoto 604-8511 (JP)
(74) Representative: Müller-Boré & Partner Patentanwälte PartG mbB
(86) International application number: PCT/JP2022/024863
(87) International publication number: WO 2023/282061

(57) **Abstract**

A microorganism mass spectrometry method includes: forming (11), on a sample plate, a mixed crystal of a matrix material and a microorganism sample which is a sample including a test microorganism or a component derived from the test microorganism; performing, in a mass spectrometer for performing analysis through matrix-assisted laser desorption/ionization mass spectrometry, a first laser irradiation (12) in which a predetermined micro region on the mixed crystal is irradiated with a laser beam for a predetermined number of times; performing, in the mass spectrometer, a second laser irradiation (13) in which the micro region or a predetermined region in the micro region is irradiated with the laser beam for a predetermined number of times; and generating (14) mass spectrometry data indicating a mass spectrometry result for the mixed crystal, on the basis of ion detection signals acquired by the mass spectrometry device during the second laser irradiation. This makes it possible to detect microorganism-derived ions with sufficiently high signal intensity.

## Description

### TECHNICAL FIELD

The present invention relates to a microorganism mass spectrometry method.

### BACKGROUND ART

A matrix-assisted laser desorption/ionization (MALDI) method, which is one of ionization methods in mass spectrometry, is a method for ionizing an analysis target substance by mixing a matrix substance that is likely to absorb laser light and is likely to be ionized with a substance to be analyzed, and irradiating the mixture with laser light, in order to analyze a substance that is difficult to absorb laser light or a substance that is likely to be damaged by laser light, such as a protein. Generally, a matrix substance is mixed with an analysis target substance as a solution, and the matrix solution incorporates the analysis target substance. Then, the solvent in the solution is vaporized and dried, and a crystal (mixed crystal of the sample and the matrix substance) containing the analysis target substance is formed. When the formed crystal is irradiated with laser light, the matrix substance absorbs energy of the laser light and is rapidly heated and vaporized. At that time, the analysis target substance is vaporized together with the matrix substance, and the analysis target substance is ionized in the process.

A mass spectrometer (MALDI-MS) using the MALDI method has been widely used in the field of bioscience since it enables an analysis of macromolecule compounds such as proteins without significantly dissociating them, and furthermore, since it is suitable also for an analysis of very small amount (microanalysis). One of applications of MALDI-MS in the field of bioscience is the identification of microorganisms using MALDI-MS (see, for example, Non Patent Literature 1). This is a method of simply identifying microorganisms on the basis of mass spectral patterns obtained using test microorganisms, which enables to obtain analysis results in a short time, thus it is possible to easily and quickly identify the microorganisms.

### CITATION LIST

### NON PATENT LITERATURE

Non Patent Literature 1: "AXIMA Microorganism Identification System Simple User Guide", Shimadzu Corporation, April 6, 2017

### SUMMARY OF INVENTION

### TECHNICAL PROBLEM

However, when microorganisms are analyzed using MALDI-MS as described above, the signal intensity of ions derived from test microorganisms is sometimes too low, and microorganism identification based on the mass spectral patterns as described above may not be appropriately performed.

The present invention has been made in view of the above points, and an object of the present invention is to enable microorganism-derived ions to be detected with sufficiently high signal intensity in mass spectrometry of microorganisms using MALDI-MS.

### SOLUTION TO PROBLEM

A microorganism mass spectrometry method according to the present invention, which has been made to solve the above problem, includes:
forming, on a sample plate, a mixed crystal of a matrix material and a microorganism sample which is a sample including a test microorganism or a component derived from the test microorganism;
performing, in a mass spectrometer for performing analysis through matrix-assisted laser desorption/ionization mass spectrometry, a first laser irradiation in which a predetermined micro region on the mixed crystal is irradiated with a laser beam for a predetermined number of times;
performing, in the mass spectrometer, a second laser irradiation in which the micro region or a predetermined region in the micro region is irradiated with the laser beam for a predetermined number of times; and
generating mass spectrometry data indicating a mass spectrometry result for the mixed crystal, on the basis of ion detection signals acquired by the mass spectrometry device during the second laser irradiation.

### ADVANTAGEOUS EFFECTS OF INVENTION

According to the microorganism mass spectrometry method having the above configuration according to the present invention, microorganism-derived ions can be detected with sufficiently high signal intensity in microorganism analysis using MALDI-MS.

### BRIEF DESCRIPTION OF DRAWINGS

[Fig. 1] Fig. 1 is a schematic configuration diagram of a microorganism analysis system according to an embodiment of the present invention.
[Fig. 2] Fig. 2 is a flowchart showing a procedure of microorganism analysis in the same embodiment.
[Fig. 3] Fig. 3 is a graph showing transition of protein peak intensity in First Test Example.
[Fig. 4] Fig. 4 is a graph showing transition of protein peak intensity in Second Test Example.
[Fig. 5] Fig. 5 is a comparison diagram of mass spectra of a first analysis and a second analysis in Third Test Example.
[Fig. 6] Fig. 6 is a diagram showing changes in mass spectra and microorganism identification results depending on the number of laser irradiation before data acquisition in Fourth Test Example.
[Fig. 7] Fig. 7 is a graph showing transition of protein peak intensity in Fifth Test Example.
[Fig. 8] Fig. 8 is a comparison diagram of mass spectra of a first analysis and a second analysis in Fifth Test Example.
[Fig. 9] Fig. 9 is a graph showing transition of protein peak intensity in Sixth Test Example.
[Fig. 10] Fig. 10 is a comparison diagram of mass spectra of a first analysis and a second analysis in Sixth Test Example.

### DESCRIPTION OF EMBODIMENTS

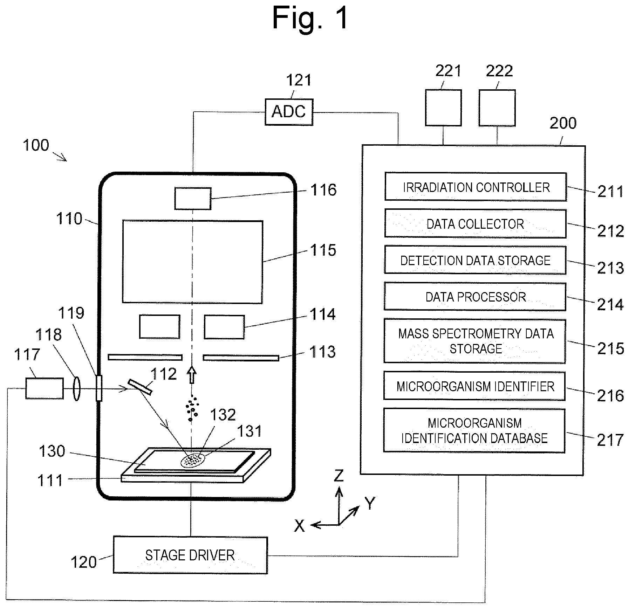

Embodiments of the present invention are described hereinafter, with reference to the drawings. Fig. 1 is a schematic configuration diagram of a microorganism analysis system according to an embodiment of the present invention.

This system includes a mass spectrometer 100 and a controller/processor 200 that controls operations of the mass spectrometer 100 and processes signals output from the mass spectrometer 100.

The mass spectrometer 100 is a mass spectrometer (MALDI-MS) using a matrix-assisted laser desorption/ionization (MALDI) method as a sample ionization method. In the mass spectrometer 100, a sample stage 111, a reflection optical system 112, an extraction electrode 113, an ion transport optical system 114, a mass separator 115, an ion detector 116, and the like are disposed inside a vacuum chamber 110 evacuated by a vacuum pump (not illustrated). A laser light source 117, a condensing optical system 118, and the like are disposed outside the vacuum chamber 110.

A sample plate 130 used in the MALDI method is placed on the upper surface of the sample stage 111. A plurality of wells 131 (regions on which samples are placed) are provided on the upper surface of the sample plate 130, and a mixed crystal 132 of a sample containing a test microorganism or a component derived from the test microorganism (hereinafter referred to as a "microorganism sample") and a matrix substance is held in each of the wells 131. In Fig. 1, only one well 131 is shown for simplification. The sample stage 111 is movable in two axial directions of an X-axis and a Y-axis respectively parallel to the upper surface of the sample stage 111 and orthogonal to each other by a stage driver 120 including a stepping motor (not illustrated).

The laser light source 117 emits laser light, which is ultraviolet light, in a pulsed manner. The laser light emitted from the laser light source 117 is narrowed to a small diameter by the condensing optical system 118, and then enters the vacuum chamber 110 through a window portion 119 that is provided on a wall of the vacuum chamber 110 and is capable of transmitting ultraviolet rays. The laser light entering the vacuum chamber 110 is reflected by the reflection optical system 112 and enters the mixed crystal 132 on the sample plate 130. That is, the laser light source 117, the condensing optical system 118, the window portion 119, and the reflection optical system 112 correspond to a laser irradiator in the present invention. Since the condensing position of the laser light is fixed, when the sample stage 111 is moved in the X-Y plane by the stage driver 120, the position on the mixed crystal 132 where the laser light strikes, that is, a micro region (hereinafter referred to as "irradiation target region") to be irradiated with the laser light changes.

The extraction electrode 113 is a plate-like electrode having an opening for allowing ions to pass through at the center, and is arranged in the vicinity of the sample stage 111 so as to face the upper surface of the sample stage 111. A voltage is applied between the extraction electrode 113 and the sample stage 111 by a high-voltage power supply (not illustrated), and ions generated from the mixed crystal 132 by irradiation with the laser beam are accelerated in a Z-axis direction orthogonal to the X-axis and the Y-axis by an electric field formed by the application of the voltage.

The ion transport optical system 114 for transporting ions while converging the ions by the action of the electric field is installed behind the extraction electrode 113, and a mass separator 115 for separating the ions according to m/z and an ion detector 116 for detecting the separated ions are installed further behind the ion transport optical system 114. Detection signals by the ion detector 116 are converted into digital data by an analog-to-digital converter (ADC) 121 and input to the controller/processor 200. Hereinafter, the digital data is referred to as "detection data". For example, an electrostatic lens, a multipole type highfrequency ion guide, or a combination of the electrostatic lens and the multipole type radiofrequency ion guide is used as the ion transport optical system 114. As the mass separator 115, for example, various types such as an ion trap type, a quadrupole type, a double convergence type, a time-of-flight type, and a magnetic field sector type can be used.

The controller/processor 200 includes an irradiation controller 211, a data collector 212, a detection data storage 213, a data processor 214, a mass spectrometry data storage 215, a microorganism identifier 216, and a microorganism identification database 217.

The irradiation controller 211 (corresponding to the controller in the present invention) controls operations of the stage driver 120 and the laser light source 117. Although signal lines are not illustrated in Fig. 1 to avoid complexity, the controller/processor 200 also controls operations of the extraction electrode 113, the ion transport optical system 114, the mass separator 115, the ion detector 116, the vacuum pump (not illustrated), and the like. The data collector 212 collects detection data input from the ion detector 116 along with the execution of the analysis and stores the detection data in the detection data storage 213. The data processor 214 generates mass spectrometry data (for example, profile data, a mass spectrum, or a peak list to be described later) representing an analysis result by the mass spectrometer 100 on the basis of the detection data stored in the detection data storage 213. The mass spectrometry data storage 215 stores the mass spectrometry data generated by the data processor 214.

The microorganism identifier 216 identifies test microorganisms by collating the mass spectrometry data generated by the data processor 214 with the microorganism identification database 217.

Mass lists related to a plurality of known microorganisms are registered in the microorganism identification database 217. The mass lists are lists listing m/z of ions detected when cells of each known microorganism are subjected to mass spectrometry, and includes at least information (classification information) of a classification group (family, genus, species, strain, or the like) to which the known microorganism belongs, in addition to the value of m/z.

An entity of the controller/processor 200 is a computer such as a personal computer or a workstation, and the computer includes a CPU, a memory, and a mass storage device including a hard disk, a flash memory, or the like. An input unit 221 including a keyboard, a mouse, or the like and a display 222 including a display device such as a liquid crystal display are connected to the controller/processor 200. The irradiation controller 211, the data collector 212, the data processor 214, and the microorganism identifier 216 described above are embodied by the CPU executing dedicated control and processing software installed in advance in this computer. Note that the detection data storage 213, the mass spectrometry data storage 215, and the microorganism identification database 217 are constituted by a mass storage device built in the computer or directly connected to the computer, or for example, a storage device existing on another computer system accessible from the computer via the Internet or the like, for example, a storage device in cloud computing or the like may be used.

In addition, in the system according to the present embodiment, a function of the irradiation controller 211 and functions of the data processor 214 and the microorganism identifier 216 can be shared by different computers. Specifically, for example, it is conceivable to assign the function of the irradiation controller 211 to one computer directly connected to the mass spectrometer 100, and assign the function of the data processor 214 and the function of the microorganism identifier 216 to another computer connected to the computer directly or via a network such as the Internet. Furthermore, the function of the data processor 214 and the function of the microorganism identifier 216 may be shared by different computers.

Next, characteristics of the microorganism analysis method performed using the system according to the present embodiment will be described.

Generally, in the analysis by MALDI-MS, a plurality of (for example, 100) irradiation target regions are set for one well, and a process of generating ions by laser light irradiation and separating and detecting the generated ions is repeatedly executed for each irradiation target region a plurality of times (for example, about 5 to 10 times). Then, one profile data is created by summing up ion detection signals obtained for one irradiation target region. Therefore, for one well, the same number of pieces of profile data as the number of irradiation target regions set on the well is obtained. Here, the profile data represents a waveform of the detection signals continuously transmitted from the ion detector provided in the mass spectrometer, where a horizontal axis represents time (or m/z) and a vertical axis represents ionic intensity. Thereafter, one mass spectrum (representative mass spectrum) related to the mixed crystal formed on one well is created by taking the average or the like of the profile data generated for one well, and further, a list (peak list) or the like showing the m/z value representing the centroid position (or center position) of each peak included in the representative mass spectrum and the area value of each peak is created.

However, when a microorganism sample is analyzed by the conventional general MALDI-MS, the signal intensity of ions derived from the microorganism sample may be low. As a result of intensive studies, as a factor of the above, the present inventor came to consider the influence of impurities taken in from the atmosphere at the time of forming a mixed crystal of a sample and a matrix.

That is, ionization in the analysis by general MALDI-MS as described above is limited to a very shallow region near the surface in the mixed crystal of the sample and the matrix substance. However, it is considered that this region contains a large amount of impurities (for example, minerals) taken in from the atmosphere in the process of drying the mixed solution of the sample and the matrix solution to form the mixed crystal. Therefore, it is expected that ionization efficiency of components such as microorganism-derived proteins is reduced due to suppression of ionization (ion suppression) by the impurities.

Therefore, in the system according to the present embodiment, the surface layer portion of the mixed crystal is removed in advance by irradiating the mixed crystal 132 to be analyzed with laser light before performing mass spectrometry (hereinafter, sometimes referred to as "main analysis") for mass spectrum creation. As a result, in the main analysis, a region of the mixed crystal 132 in which the content of impurities taken in from the atmosphere is small can be ionized, so that ion suppression by the impurities can be reduced, and microorganism-derived ions can be detected with sufficiently high signal intensity.

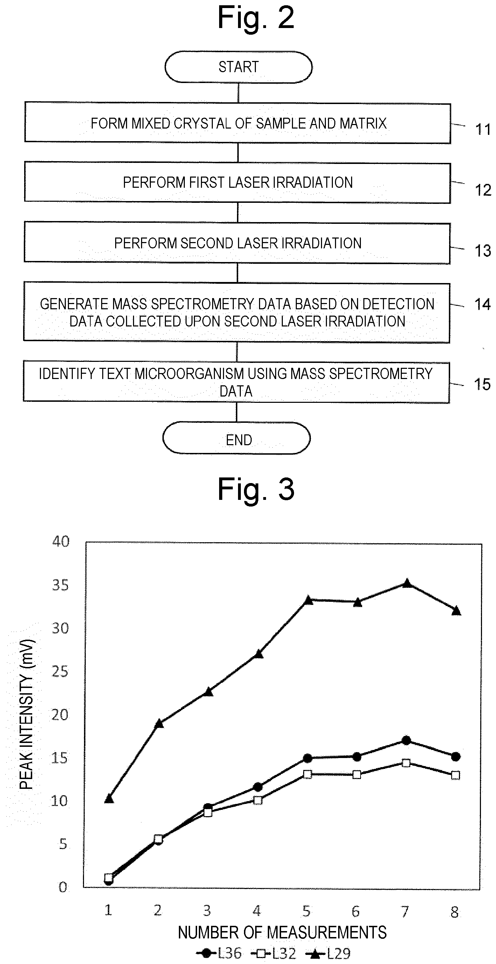

Hereinafter, details of such a microorganism analysis method will be described with reference to the flowchart of Fig. 2.

First, a user of the system according to the present embodiment (that is, an analyst) forms a mixed crystal 132 of a microorganism sample and a matrix substance in a predetermined well 131 on the sample plate 130 (step 11). Specifically, for example, a solution of a matrix substance (matrix solution) and a microorganism sample are mixed in a microtube such as an Eppendorf tube, and the mixture is added dropwise onto the well 131 and dried to form the mixed crystal 132 of the sample and the matrix substance. Alternatively, the mixed crystal 132 may be formed by placing a microorganism sample on the well 131, then dropping a matrix solution onto the same well 131, and mixing and drying them on the well 131. However, the method for preparing the mixed crystal 132 is not limited thereto, and various known methods can be applied. Examples of the microorganism (test microorganism) to be analyzed include, but are not limited to, bacteria, yeast, and mold (filamentous fungus). When a microorganism having a hard cell wall such as gram-positive bacteria, mold, or yeast is to be analyzed, pretreatment with an organic acid such as formic acid treatment may be performed in advance before mixing the microorganism with the matrix solution. The type of the matrix substance is not particularly limited, and CHCA (α-cyano-4-hydroxycinnamic acid), SA (sinapinic acid), DHB (2,5-dihydroxybenzoic acid), or the like can be used as appropriate. As the solvent of the matrix solution, for example, a solution obtained by adding 0 to 3 vol% of trifluoroacetic acid (TFA) to an aqueous solution containing several tens vol% of an organic solvent such as acetonitrile can be used, but the solvent is not limited thereto. As the microorganism sample in the present embodiment, a microorganism cell (microbial body) itself collected from a culture medium or the like may be used, or an extract from a microorganism cell or a cell constituent such as a protein purified from the extract may be used.

Next, the user sets the sample plate 130 holding the mixed crystal 132 on the sample stage 111 of the mass spectrometer 100. Then, the number of irradiation target regions in the mixed crystal 132 in each well 131 and the position and size of each irradiation target region are set by operating the input unit 221, and the number of times of laser irradiation N₁ in a first laser irradiation and the number of times of laser irradiation N₂ in a second laser irradiation described later are set. The number, position, and size of the irradiation target regions, or the number of times of laser irradiations N₁ and N₂ may be automatically set by the controller/processor 200. Thereafter, when the user instructs the controller/processor 200 to start analysis by performing a predetermined operation from the input unit 221, vacuuming of the vacuum chamber 110 is executed by the vacuum pump, which is not illustrated. In the following description, for the sake of simplicity, it is assumed that only the mixed crystal 132 formed in one well 131 on the sample plate 130 is analyzed by the system of the present embodiment.

When the vacuuming is completed, a first laser irradiation is performed on each irradiation target region set on the mixed crystal 132 (step 12). Specifically, first, the irradiation controller 211 controls the stage driver 120 to move the sample stage 111, and moves the first irradiation target region on the mixed crystal 132 to the laser beam condensing position. Then, the irradiation controller 211 controls the laser light source 117 to emit laser light a predetermined number of times N₁ (for example, about 5 to 25 times). The frequency of the laser at this time is, for example, 20 Hz to 200 Hz, preferably about 40 to 60 Hz. The laser light emitted from the laser light source 117 is narrowed to a small diameter by the condensing optical system 118, and then enters the first irradiation target region via the window portion 119 and the reflection optical system 112. By the irradiation of the laser beam, a part of the mixed crystal 132 in the first irradiation target region is desorbed from the surface layer portion of the crystal and ionized. The ions are extracted from the vicinity of the well 131 by the extraction electrode 113 and introduced into the mass separator 115 via the ion transport optical system 114. Then, the ions are separated according to m/z and then detected by the ion detector 116. However, at this time, the detection data input from the ion detector 116 to the controller/processor 200 via the ADC is not stored in the detection data storage 213 (that is, the data collector 212 does not collect data during execution of the first laser irradiation).

When first laser irradiation to the first irradiation target region is completed, the irradiation controller 211 controls the stage driver 120 to move the sample stage 111, moves the second irradiation target region on the well 131 to the condensing position of the laser beam, and executes the first laser irradiation in the same manner as described above. Thereafter, similarly, the first laser irradiation is sequentially performed on the third and subsequent irradiation target regions.

As described above, when the first laser irradiation is completed for all the irradiation target areas, subsequently, a second laser irradiation is executed for each irradiation target area (step 13), and the output signals (detection data) from the ion detector 116 at that time is collected by the data collector 212. Specifically, first, under the control of the irradiation controller 211, the stage driver 120 moves the sample stage 111, and moves the first irradiation target region on the well 131 to the laser beam condensing position again. Then, under the control of irradiation controller 211, the laser light source 117 emits laser light a predetermined number of times N₂ (for example, about 5 to 25 times). The frequency of laser irradiation at this time is also about 40 to 60 Hz, for example. At this time, every time the laser beam is emitted once, a portion of the mixed crystal 132 in the first irradiation target region is desorbed from the surface of the crystal and ionized, and is introduced into the mass separator 115 through the ion transport optical system 114. The ions introduced into the mass separator 115 are separated according to m/z and then detected by the ion detector 116, and the detection signals output from the ion detector 116 is converted into digital data (detection data) by the ADC 121 and then input to the controller/processor 200. The input detection data is collected by the data collector 212 and stored in the detection data storage 213.

When the second laser irradiation to the first irradiation target region and the collection of the detection data are completed, the irradiation controller 211 controls the stage driver 120 to move the sample stage 111, moves the second irradiation target region on the well 131 to the condensing position of the laser beam, and executes the second laser irradiation and the collection of the detection data in the same manner as described above. Thereafter, similarly, the second laser irradiation and the collection of the detection data are sequentially performed on the third and subsequent irradiation target regions.

As described above, when the second laser irradiation and the collection of the detection data are completed for all the irradiation target regions, the data processor 214 reads the detection data collected during execution of the second laser irradiation from the detection data storage 213, and generates mass spectrometry data indicating a result of mass spectrometry for the mixed crystal 132 (step 14). Specifically, first, one profile data is generated for each irradiation target region by summing up N₂ pieces of detection data collected during the second laser irradiation for each irradiation target region. As a result, the same number of pieces of profile data as the number of the irradiation target region are obtained. Subsequently, an average value (or a median value or a mode value) of the values of the ionic intensity at each m/z of the profile data is obtained, and a mass spectrum (representative mass spectrum) in which the vertical axis is the average value (or the median value or the mode value) and the horizontal axis is m/z is created. Furthermore, a peak list is created by listing m/z values representing the centroid position (or center position) of each peak included in the representative mass spectrum and the area value (or height) of each peak. The representative mass spectrum and the peak list created as described above are stored in the mass spectrometry data storage 215 as a final analysis result (mass spectrometry data) for the microorganism sample contained in the mixed crystal 132 in the well 131.

Thereafter, the microorganism identifier 216 identifies the test microorganism on the basis of the mass spectrometry data (that is, the representative mass spectrum or the peak list) created as described above (step 15). Specifically, for example, the microorganism identifier 216 reads the peak list (that is, the peak list of the test microorganism) for the microorganism sample generated in step 14 from the mass spectrometry data storage 215, and collates the read peak list with the mass list of each known microorganism stored in the microorganism identification database 217. Then, a mass list having an m/z pattern similar to the peak list of the test microorganism, for example, a mass list containing a large amount of m/z matching with m/z of each peak in the peak list of the test microorganism within a predetermined error range is extracted. Subsequently, the microorganism identifier 216 refers to classification information stored in the microorganism identification database 217 in association with the extracted mass list to specify a classification group (family, genus, species, strain, or the like) to which a known microorganism corresponding to the mass list belongs. Then, the specified classification group is displayed on the display 222 as a classification group to which the test microorganism is estimated to belong.

As described above, here, for the sake of simplicity, the process of steps 12 to 15 is performed only for the mixed crystal 132 formed in one well 131 on the sample plate 130, but usually, the first laser irradiation (step 12) and the second laser irradiation (step 13) described above are performed for each of the mixed crystals 132 formed in the plurality of wells 131 on the sample plate 130, and thereafter, mass spectrometry data for each of the mixed crystals 132 is created (step 14), and the test microorganism corresponding to each of the mixed crystals 132 is identified (step 15).

In the above example, the data collector 212 does not collect data during execution of the first laser irradiation (step 12), but instead, the data collector 212 may collect data (that is, the detection data output from the ion detector 116 is stored in the detection data storage 213) also during execution of the first laser irradiation. However, even in this case, at the time of generating the mass spectrometry data in step 14, the detection data collected during execution of the first laser irradiation is not used, and the mass spectrometry data is generated on the basis of the detection data collected during execution of the second laser irradiation (step 13).

In the above example, the ions generated from the mixed crystal 132 are separated by the mass separator 115 and detected by the ion detector 116 in both the first laser irradiation and the second laser irradiation. However, in the first laser irradiation, such separation and detection of ions may not necessarily be performed.

Furthermore, in the above example, the second laser irradiation is performed for each irradiation target region after the first laser irradiation is completed for all the irradiation target regions on the mixed crystal 132, but the present invention is not limited thereto, and the first laser irradiation and the second laser irradiation may be performed for the next irradiation target region after both the first laser irradiation and the second laser irradiation for one irradiation target region (that is, (Ni + N₂) times of laser irradiation for the irradiation target region) are completed. Also in this case, during execution of the first laser irradiation (that is, the first to Ni-th laser irradiation in each irradiation target region), the separation and detection of the ions generated from the mixed crystal 132 and the collection data of the detection data output from the ion detector 116 may be performed, or only the separation and detection of the ions may be performed and the data collection may not be performed. Alternatively, any of ion separation, detection, and data collection may not be performed during execution of the first laser irradiation. In either case, when the mass spectrometry data is generated in step 14, the mass spectrometry data is generated using the detection data collected by the second laser irradiation (that is, the (Ni + 1)-th to N₂-th laser irradiation) on each irradiation target region.

According to the microorganism analysis system of the present embodiment, by performing laser irradiation on each of the irradiation target regions on the mixed crystal before performing the main analysis (mass spectrometry for mass spectrum creation), it is possible to obtain profile data in which ionization of components derived from microorganisms more reliably occurs in the main analysis. As a result, it is expected that a mass spectrum having a high peak intensity of a component derived from a microorganism can be obtained, and a microorganism that has a low peak intensity and cannot be appropriately identified by a conventional method can also be identified.

By analyzing a large number of known microorganisms using the microorganism analysis system according to the present embodiment, it is also possible to create a database in which a high-quality mass list (that is, a mass list containing a large amount of m/z relating to components derived from the microorganism and having a small amount of m/z relating to other undesirable components) regarding each known microorganism is recorded. When such a database is created, a peak list of each known microorganism is generated by executing steps 11 to 14 described above using each known microorganism as a test microorganism, and a peak in a predetermined m/z range is extracted from each peak list. At this time, by setting the m/z range to about 2,000 to 35,000, it is possible to mainly extract a protein-derived peak. Furthermore, by extracting only peaks whose height (relative intensity) is equal to or greater than a predetermined threshold, undesirable peaks (noise) is excluded. Since the ribosomal protein group is expressed in a large amount in the cell, most of the above-described extracted m/z also can be derived from the ribosomal protein by appropriately setting the threshold. Then, a mass list that is a list in which m/z of the peaks extracted as described above is described is created, and the mass list and classification information of known microorganisms corresponding to the mass list are registered in the database in association with each other.

As described above, it is possible to construct a database in which a high-quality mass list related to a plurality of known microorganisms is recorded. In addition, the database constructed in advance in this manner may be used as the microorganism identification database 217 in the system according to the present embodiment. As a result, higher identification accuracy can be achieved when the microorganism is identified through the steps 11 to 15 using the unknown microorganism as the test microorganism.

Although the embodiment of the present invention is described with specific examples, the present invention is not limited to such an embodiment, and an appropriate change in the scope of the present invention is acceptable. For example, in the above example, the second laser irradiation is performed on the same area as the area where the first laser irradiation is performed (that is, the above-described irradiation target region), but the second laser irradiation may be performed on a narrower region in a micro region where the first laser irradiation is performed. In that case, the user may set in advance the micro region where the first laser irradiation is performed and the micro region where the second laser irradiation is performed (the above-described "narrower region"), or the user may set the micro region where the first laser irradiation is performed so that the narrower region in the micro region is automatically set as the area where the second laser irradiation is performed. Alternatively, the controller/processor 200 may automatically set both the region where the first laser irradiation is performed and the region where the second laser irradiation is performed.

Hereinafter, test examples performed to confirm the effect of the spectrometry method of the present invention will be described.

### [First Test Example]

A matrix solution was prepared by dissolving 10 mg/mL CHCA in 50% acetonitrile 1% trifluoroacetic acid aqueous solution. E. coli and the matrix solution were mixed so that the optical density (OD) was about 1, and 1 µL of the resulting mixed solution was added dropwise onto a sample plate. Then, this was dried to form a mixed crystal of the sample and the matrix on the sample plate. The sample plate was set in MALDI-MS, and each of 100 irradiation target regions set on the mixed crystal was irradiated with laser at 50 Hz for 5 shots. Then, a total of 100 pieces of profile data were acquired by summing up detection signals output from the ion detector in association with laser irradiation of each irradiation target region. The measurement of the same mixed crystal (that is, laser irradiation of 5 shots for each of the 100 irradiation target regions) was repeated 8 times without changing the laser irradiation conditions, and the average value of the peak intensities (mV) of the ribosomal proteins L36, L32, and L29 on the 100 pieces of profile data in each measurement was plotted, and the result is shown in Fig. 3. As shown in the graph, the peak intensities of the three types of ribosomal proteins showed a significant increasing tendency from the first measurement to the fifth measurement, and a gradual increasing tendency from the fifth measurement to the eighth measurement. From this result, it was shown that removing the surface layer portion of the mixed crystal by laser irradiation is effective for obtaining high peak intensity in microorganism analysis by MALDI-MS.

### [Second Test Example]

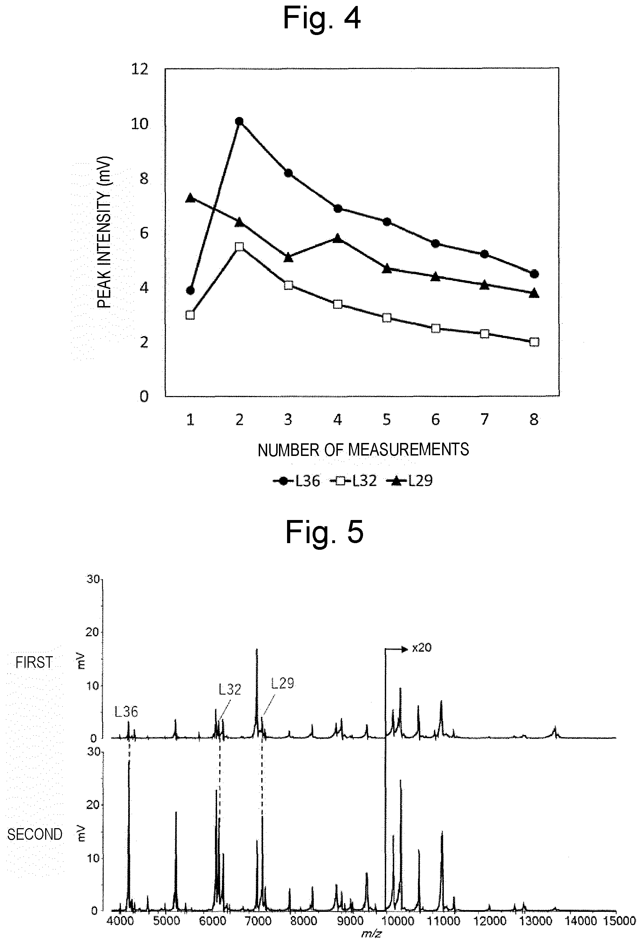

A matrix solution was prepared by dissolving 15 mg/mL SA in 50% acetonitrile 1% trifluoroacetic acid aqueous solution. E. coli and the matrix solution were mixed so that the OD was about 1, and 1 µL of the resulting mixed solution was added dropwise onto a sample plate. Then, this was dried to form a mixed crystal of the sample and the matrix substance on the sample plate. The sample plate was set in MALDI-MS, and the mixed crystal was repeatedly measured 8 times in the same manner as in First Test Example. The average value of the peak intensities (mV) of the ribosomal proteins L36, L32, and L29 on the 100 pieces of profile data in each measurement was plotted, and the result is shown in Fig. 4. As shown in the graph, the intensities of the peaks of L36 and L32 were two times or more higher in the second measurement than in the first measurement, and peaks were observed at higher intensities than in the first measurement up to the fourth to fifth measurements. From this result, it was shown that removing the surface of the mixed crystal by laser irradiation is effective for obtaining high peak intensity in microorganism analysis by MALDI-MS.

### [Third Test Example]

A matrix solution was prepared by dissolving 10 mg/mL CHCA in 50% acetonitrile 1% trifluoroacetic acid aqueous solution. E. coli and the matrix solution were mixed so that the OD was about 1, and 1 µL of the resulting mixed solution was added dropwise onto a sample plate. Then, this was dried to form a mixed crystal of the sample and the matrix on the sample plate. The sample plate was set in MALDI-MS, and each of 100 irradiation target regions set on the mixed crystal was irradiated with laser at 50 Hz for 5 shots. Then, a total of 100 pieces of profile data were acquired by summing up detection signals output from the ion detector in association with laser irradiation of each irradiation target region, and a representative mass spectrum was created by averaging the profile data. Thereafter, measurement of the same mixed crystal (that is, laser irradiation of 5 shots for each of the 100 irradiation target regions) was performed again without changing the laser irradiation conditions, and 100 pieces of profile data were acquired and a representative mass spectrum was created in the same manner. A representative mass spectrum in each measurement is shown in Fig. 5. From this graph, it can be seen that the representative mass spectrum in the second measurement has a higher peak intensity as a whole than the representative mass spectrum in the first measurement. In addition, in the mass spectrum in Fig. 5, the resolution of the peaks of three types of ribosomal proteins L36, L32, and L29 was as follows: L36: first: 327; second: 316; L32: first: 409; second: 442; and L29: first: 383; second: 498; and a mass spectrum having a higher mass resolution was obtained in the second measurement except for L36.

### [Fourth Test Example]

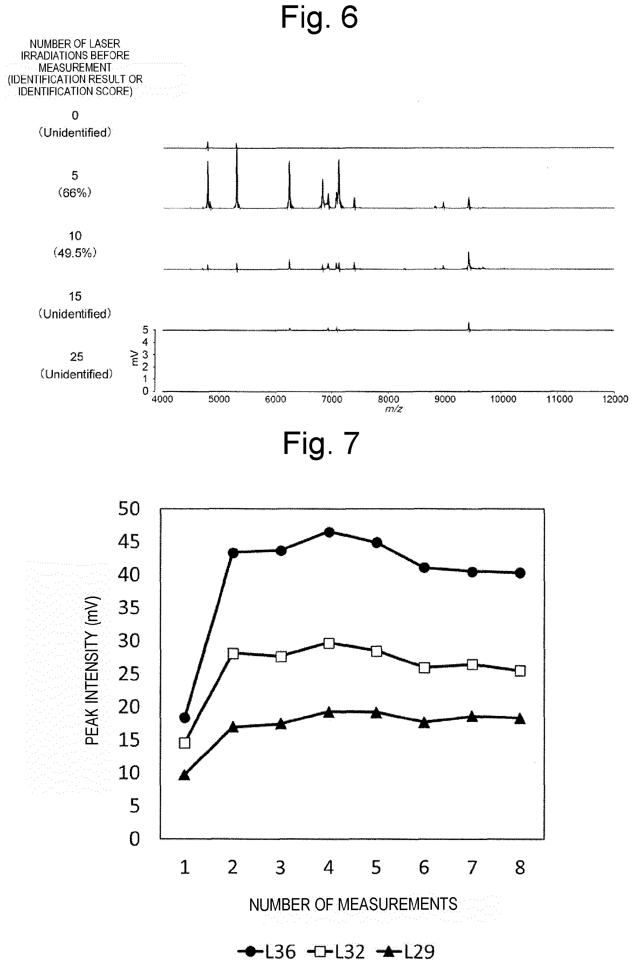

A matrix solution was prepared by dissolving 10 mg/mL CHCA in 50% acetonitrile 1% trifluoroacetic acid aqueous solution. Brevundimonas diminuta, which is a gram-negative bacterium, and the matrix solution were mixed so that the OD was about 1, and 1 µL of the resulting mixed solution was added dropwise onto a sample plate. Then, this was dried to form a mixed crystal of the sample and the matrix substance on the sample plate. The sample plate was set in MALDI-MS, and first, laser irradiation without taking in detection signals from the ion detector was performed 0 times, 5 times, 10 times, 15 times, or 25 times for each of 100 irradiation target regions set on the mixed crystal. Thereafter, laser irradiation was performed 5 times for each of the same 100 irradiation target regions, and a total of 100 pieces of profile data were acquired by summing up detection signals output from the ion detector during the irradiation, and a representative mass spectrum was created by averaging the profile data. The representative mass spectrum obtained above and the result of microorganism identification using the representative mass spectrum are shown in Fig. 6. As shown in the graph, in the case where laser irradiation before data acquisition (that is, before main analysis) was performed 0 times, two weak peaks were detected, and no microorganism was identified. In the case where laser irradiation was performed 5 times before data acquisition, a large number of high intensity peaks were detected and identified as B. diminuta with an identification score of 66%. Also in the case where laser irradiation was performed 10 times before data acquisition, a large number of high intensity peaks were detected and identified as B. diminuta with an identification score of 49.5%. In the case where laser irradiation was performed 15 times before data acquisition, only a few weak peaks were detected, and in the case where laser irradiation was performed 25 times before data acquisition, no peak was detected at all, and no microorganism was identified.

### [Fifth Test Example]

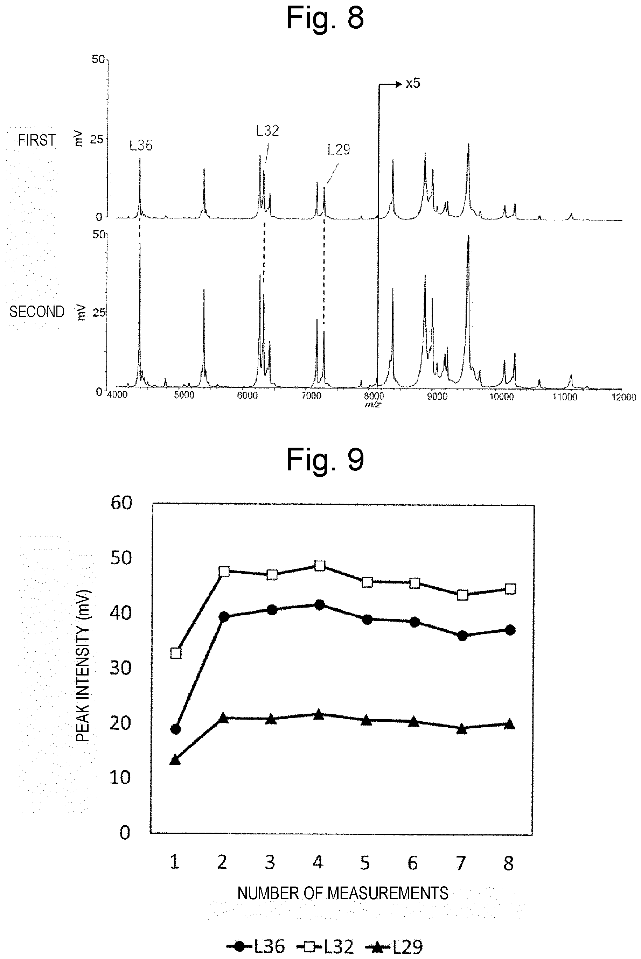

0.5 µL of a 25% aqueous formic acid solution was added dropwise onto the cells of lactic acid bacteria applied to the sample plate, and the cells and formic acid were mixed by pipetting using a micropipette to lyse the cells. This was dried, then 1 µL of 10 mg/mL CHCA (50% acetonitrile 1% trifluoroacetic acid aqueous solution) was added dropwise, and the resulting was mixed with cells subjected to lysis treatment with formic acid by pipetting. Then, this was dried to form a mixed crystal of the sample and the matrix substance on the sample plate. The sample plate was set in MALDI-MS, and the mixed crystal was repeatedly measured 8 times in the same manner as in First Test Example. The average value of the peak intensities (mV) of the ribosomal proteins L36, L32, and L29 on the profile data obtained in each measurement was plotted, and the result is shown in Fig. 7. As shown in the graph, the intensities of peaks derived from the three types of proteins significantly increased from the first measurement to the fifth measurement, and gradually increased from the fifth measurement to the eighth measurement. Fig. 8 shows a representative mass spectrum in the first measurement and a representative mass spectrum in the second measurement. From this graph, it can be seen that the representative mass spectrum in the second measurement has a higher peak intensity as a whole than the representative mass spectrum in the first measurement. In addition, the resolution of the peaks of the three types of ribosomal proteins L36, L32, and L29 was as follows: L36: first: 358; second: 375; L32: first: 334; second: 365; and L29: first: 469; second: 488; and the mass resolution was higher in the second measurement.

### [Sixth Test Example]

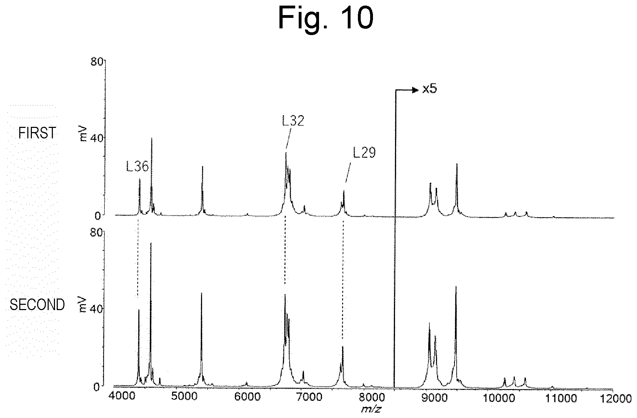

1 µL of CHCA (50% acetonitrile 1% trifluoroacetic acid aqueous solution) of 10 mg/mL was added dropwise to cells of E. coli applied to a sample plate, and mixed with the cells by pipetting using a micropipette. Then, this was dried to form a mixed crystal of the sample and the matrix substance on the sample plate. The sample plate was set in MALDI-MS, and the mixed crystal was repeatedly measured 8 times in the same manner as in First Test Example. The average value of the peak intensities (mV) of the ribosomal proteins L36, L32, and L29 on the profile data obtained in each measurement was plotted, and the result is shown in Fig. 9. As shown in the graph, the intensities of peaks derived from the three types of proteins greatly increased from the first measurement to the fifth measurement, and showed a gradual increase from the fifth measurement to the eighth measurement. Fig. 10 shows a representative mass spectrum in the first measurement and a representative mass spectrum in the second measurement. From this graph, it can be seen that the representative mass spectrum in the second measurement has a higher peak intensity as a whole than the representative mass spectrum in the first measurement. In addition, the resolution of the peaks of the three types of ribosomal proteins L36, L32, and L29 was as follows: L36: first: 317; second: 320; L32: first: 385, second: 399, and L29: first: 503; and second: 512; and the mass resolution was high in the second measurement.

### [Various Modes]

A person skilled in the art can understand that the previously described illustrative embodiments are specific examples of the following modes of the present invention.

(Clause 1) A microorganism mass spectrometry method according to one mode of the present invention includes:
forming, on a sample plate, a mixed crystal of a matrix material and a microorganism sample which is a sample including a test microorganism or a component derived from the test microorganism;
performing, in a mass spectrometer for performing analysis through matrix-assisted laser desorption/ionization mass spectrometry, a first laser irradiation in which a predetermined micro region on the mixed crystal is irradiated with a laser for a predetermined number of times;
performing, in the mass spectrometer, a second laser irradiation in which the micro region or a predetermined region in the micro region is irradiated with the laser for a predetermined number of times; and
generating mass spectrometry data indicating a mass spectrometry result for the mixed crystal, on a basis of ion detection signals acquired by the mass spectrometry device during the second laser irradiation.

In the microorganism mass spectrometry method according to Clause 1, it is possible to perform mass spectrometry by matrix-assisted laser desorption/ionization mass spectrometry for the remaining region after removing a surface layer portion having a high content of impurities (for example, mineral content) taken in from the atmosphere from the mixed crystal of the sample and the matrix substance. Therefore, in the mass spectrometry, ion suppression by the impurities can be reduced, and microorganism-derived ions can be detected with sufficiently high signal intensity.

(Clause 2) A microorganism identification method according to a mode of the present invention includes performing identification of the test microorganism by collating the mass spectrometry data generated by the microorganism mass spectrometry method according to Clause 1 with a database storing mass spectrometry results regarding a plurality of known microorganisms.

In the microorganism identification method according to Clause 2, since identification of a test microorganism is performed using mass spectrometry data having high signal intensity of microorganism-derived ions, which is generated by the method according to item 1, it is possible to identify a microorganism with higher accuracy than before.

(Clause 3) A database creation method according to a mode of the present invention includes:
performing the microorganism mass spectrometry method according to Clause 1 on a plurality of known microorganisms using the known microorganisms as the test microorganisms; and
creating a database in which the mass spectrometry data generated for each of the plurality of known microorganisms and classification information for each of the known microorganisms are recorded.

In the database creation method described in Clause 3, since the database is created by collecting mass spectrometry data for each of a plurality of known microorganisms by the method described in Clause 1, it is possible to create a high-quality database in which a large number of mass spectrometry data having high signal intensity of microorganism-derived ions are recorded.

(Clause 4) A microorganism analysis system according to a mode of the present invention is a system for performing analysis by matrix-assisted laser desorption/ionization mass spectrometry on a mixed crystal of a matrix material and a microorganism sample which is a sample including a test microorganism or a component derived from the test microorganism, the system including:
a laser irradiator configured to irradiate the mixed crystal with a laser beam in a pulsed manner;
a sample stage configured to support a sample plate holding the mixed crystal;
a stage driver configured to move the sample stage in a plane parallel to the sample plate;
a mass separator configured to separate ions generated from the mixed crystal by irradiation of the laser beam on a basis of m/z;
an ion detector configured to detect ions separated by the mass separator;
a controller configured to control the laser irradiator and the stage driver; and
a data processor configured to process detection signals from the ion detector; wherein
the controller is configured to control the laser irradiator and the stage driver so as to perform a first laser irradiation in which a predetermined micro region on the mixed crystal is irradiated with the laser beam for a predetermined number of times, and then a second laser irradiation in which the micro region or a predetermined region in the micro region is irradiated with the laser beam for a predetermined number of times, and
the data processor is configured to generate mass spectrometry data indicating a mass spectrometry result for the mixed crystal on a basis of detection signals from the ion detector during the second laser irradiation.

In the microorganism mass spectrometry method described in Clause 4, the surface layer portion having a high content of impurities taken in from the atmosphere can be removed from the mixed crystal of the sample and the matrix substance by the first laser irradiation. Then, the region below the surface layer portion is ionized by the second laser irradiation, and mass spectrometry data is created on the basis of the detection signals from the ion detector at that time, whereby ion suppression by the impurities is reduced, and mass spectrometry data having high peak intensity of components derived from microorganisms can be obtained.

### REFERENCE SIGNS LIST

- 100: Mass Spectrometer
- 111: Sample Stage
- 115: Mass Separator
- 116: Ion Detector
- 117: Laser Light Source
- 120: Stage Driver
- 130: Sample Plate
- 131: Well
- 132: Mixed Crystal
- 200: Controller/Processor
- 211: Irradiation Controller
- 212: Data Collector
- 213: Detection Data Storage
- 214: Data Processor
- 215: Mass Spectrometry Data Storage
- 216: Microorganism Identifier
- 217: Microorganism Identification Database
- 221: Input Unit
- 222: Display

## Claims

1. A microorganism mass spectrometry method comprising:
forming, on a sample plate, a mixed crystal of a matrix material and a microorganism sample which is a sample including a test microorganism or a component derived from the test microorganism;
performing, in a mass spectrometer for performing analysis through matrix-assisted laser desorption/ionization mass spectrometry, a first laser irradiation in which a predetermined micro region on the mixed crystal is irradiated with a laser beam for a predetermined number of times;
performing, in the mass spectrometer, a second laser irradiation in which the micro region or a predetermined region in the micro region is irradiated with the laser beam for a predetermined number of times; and
generating mass spectrometry data indicating a mass spectrometry result for the mixed crystal, on a basis of ion detection signals acquired by the mass spectrometry device during the second laser irradiation.

2. A microorganism identification method comprising performing identification of the test microorganism by collating the mass spectrometry data generated by the microorganism mass spectrometry method according to claim 1 with a database storing mass spectrometry results regarding a plurality of known microorganisms.

3. A database creation method comprising:
performing the microorganism mass spectrometry method according to claim 1 on a plurality of known microorganisms using the known microorganisms as the test microorganisms; and
creating a database in which the mass spectrometry data generated for each of the plurality of known microorganisms and classification information for each of the known microorganisms are recorded.

4. A microorganism analysis system for performing analysis by matrix-assisted laser desorption/ionization mass spectrometry on a mixed crystal of a matrix material and a microorganism sample which is a sample including a test microorganism or a component derived from the test microorganism, the system comprising:
a laser irradiator configured to irradiate the mixed crystal with a laser beam in a pulsed manner;
a sample stage configured to support a sample plate holding the mixed crystal;
a stage driver configured to move the sample stage in a plane parallel to the sample plate;
a mass separator configured to separate ions generated from the mixed crystal by irradiation of the laser beam on a basis of m/z;
an ion detector configured to detect ions separated by the mass separator;
a controller configured to control the laser irradiator and the stage driver; and
a data processor configured to process detection signals from the ion detector, wherein
the controller is configured to control the laser irradiator and the stage driver so as to perform a first laser irradiation in which a predetermined micro region on the mixed crystal is irradiated with the laser beam for a predetermined number of times, and then a second laser irradiation in which the micro region or a predetermined region in the micro region is irradiated with the laser beam for a predetermined number of times, and
the data processor is configured to generate mass spectrometry data indicating a mass spectrometry result for the mixed crystal on a basis of detection signals from the ion detector during the second laser irradiation.
